# EUROPEAN PATENT APPLICATION

(11) **EP 4 728 967 A1**
(43) Date of publication of application: **22.04.2026**
(21) Application number: 23942505.1
(22) Date of filing: 22.06.2023
(51) Int. Cl.: A61B 5/00, A61B 5/11, A61B 5/103, G16H 50/20, G16H 20/30, G16H 10/60

(54) **POSTURE CORRECTION METHOD AND APPARATUS THEREOF**

(30) Priority: 19.06.2023 KR 20230078307
(71) Applicant: Noogi Inc., Seoul 05510 (KR); Park, Chan Wook, Seoul 05663 (KR)
(72) Inventor: PARK, Chan Wook, Seoul 05663 (KR)
(74) Representative: Gulde & Partner
(86) International application number: PCT/KR2023/008709
(87) International publication number: WO 2024/262674

(57) **Abstract**

A posture correction method and a posture correction apparatus (100) are provided. The posture correction method may include collecting chair-sitting data of users on a chair; constructing a user-specific data cluster based on the collected chair-sitting data; constructing an Al algorithm for posture correction based on the constructed data cluster; and providing information on posture correction based on the constructed Al algorithm and real-time chair-sitting data.

The posture correction apparatus (100) may include a chair-sitting data collection unit (121) configured to collect chair-sitting data of users on a chair (300); a data cluster construction unit (122) configured to construct a user-specific data cluster based on the collected chair-sitting data; an Al algorithm construction unit (123) configured to construct an Al algorithm for posture correction based on the constructed data cluster; and a posture analysis unit (132) configured to provide information on posture correction based on the constructed Al algorithm and real-time chair-sitting data.

## Description

### Technical Field

The present invention relates to a method and an apparatus for correcting posture, and more specifically, to a method and an apparatus for correcting posture that collects chair-sitting data on various postures of a user using an ergonomically designed chair and constructs and uses an Al algorithm based on the collected data to correct the user's chair-sitting posture in real-time.

### Background

Generally, a chair is composed of a seat that supports the user's buttocks, legs that support the seat, and a backrest that supports the user's waist. Such chairs are placed in homes, offices, libraries, reading rooms, and the like, and students and office workers spend long hours sitting on the chairs.

Many users sit in wrong postures while using their cell phones, laptops, and similar devices, such as leaning their buttocks backward and leaning their heads forward. Due to this wrong posture, the rate of cervical spine disease continues to increase. For example, in Korea, more than 12 million people already suffer from cervical spine disease.

Around the world, approximately 10% of the global population is suffering from diseases such as cervical spine disease. These individual diseases affect the entire society, causing various problems such as a decrease in the productive population, an increase in the unemployment rate, a decrease in productivity, and an increase in healthcare costs.

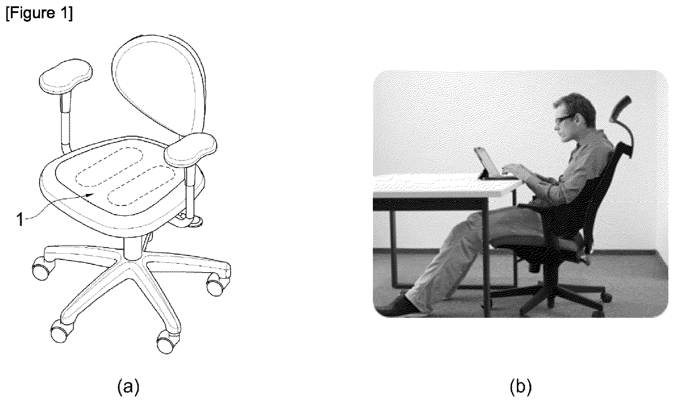

Several chairs have been proposed in the past to correct the wrong chair-sitting postures of such users and FIG. 1A illustrates such an existing posture correction chair. As illustrated in the figure, the existing posture correction chair is configured to have a pressure sensor 1 on the seat and determines the user's posture type based on the pressure value detected by the pressure sensor 1.However, since the pressure sensor 1 receives data vertically, if the user sits diagonally or crosses the seat, the pressure value cannot be accurately obtained, which is the main cause of many errors in determining whether the user has the correct posture.

Particularly, in the existing posture correction chair, if the user's buttocks and the like are evenly positioned on the seat, a pressure value classified as a good posture is obtained regardless of the posture of other body areas. For example, as illustrated in FIG. 1B, the user's buttocks are evenly in contact with the seat and the backrest is also evenly in contact with the backrest, but the neck and waist are significantly bent. However, the existing posture correction chair has a limitation that it cannot detect such a wrong posture, which can cause cervical spine disease, for example.

Therefore, to overcome the problems and limitations of the existing posture correction chairs, a need for a new type of posture correction method and apparatus that can correct the user's chair-sitting posture in real-time by collecting chair-sitting data for various postures using an ergonomically designed chair and constructing and using an Al algorithm based on the data is gradually increasing in the art. Such a need is especially increasing among many users suffering from cervical spine disease.

### Summary

### Technical Problem

The present invention has been devised to solve the problems, and an object of the present invention is to provide a posture correction method and apparatus that enables users to receive feedback on the correction of their chair-sitting postures more quickly and thereby improve their quality of life.

Another object of the present invention is to provide a posture correction method and apparatus that can assist users who are in a wrong posture to correct their postures to right postures accurately by defining the right posture that the users should pursue and defining wrong postures similar to the right posture.

Another object of the present invention is to provide a posture correction method and apparatus that can analyze differences according to user's various chair-sitting postures using a gyro sensor to identify data on specific postures that cannot be identified with a pressure sensor alone and thereby provide more user-customized posture correction information to the users.

Another object of the present invention is to provide a posture correction method and apparatus that can improve the accuracy of posture-specific analysis by confirming user's posture changes according to posture correction contents provided based on a constructed Al algorithm for posture correction and reflecting and updating the data of such posture changes back to the Al algorithm.

Another object of the present invention is to provide a posture correction method and apparatus that can further improve the accuracy of an Al posture analysis algorithm using an Al motion system based on a plurality of cameras to distinguish between right and wrong postures.

The technical problems of the present invention are not limited to the technical problems mentioned above, and other technical problems that are not mentioned can be clearly understood by those skilled in the art from the descriptions below.

### Solution to Problem

A posture correction method according to one embodiment of the present invention for solving the technical problem may include collecting chair-sitting data of users on a chair; constructing a user-specific data cluster based on the collected chair-sitting data; constructing an Al algorithm for posture correction based on the constructed data cluster; and providing information on posture correction based on the constructed Al algorithm and real-time chair-sitting data.

In addition, the posture correction method may further include analyzing improvement items of the users using the constructed Al algorithm; and updating the Al algorithm based on the analyzed improvement items.

In addition, the collecting of chair-sitting data of the users on the chair includes collecting pressure values and inclination values from a pressure sensor and a gyro sensor attached to the chair.

In addition, the collecting of the chair-sitting data of the users on the chair further includes synchronizing the collected pressure values and inclination values with Al vision data implemented by a plurality of cameras.

In addition, a computer program according to an additional embodiment of the present invention for solving the technical problem may be stored in a computer-readable recording medium that is coupled with a computer as hardware so as to be able to perform the posture correction method.

In addition, a posture correction apparatus according to another embodiment of the present invention for solving the technical problem may include a chair-sitting data collection unit configured to collect chair-sitting data of users on a chair; a data cluster construction unit configured to construct a user-specific data cluster based on the collected chair-sitting data; an Al algorithm construction unit configured to construct an Al algorithm for posture correction based on the constructed data cluster; and a posture analysis unit configured to provide information on posture correction based on the constructed Al algorithm and real-time chair-sitting data.

In addition, the posture correction apparatus may further include an Al algorithm update unit configured to analyze improvement items of the users using the constructed Al algorithm and update the Al algorithm based on the analyzed improvement items.

In addition, the chair-sitting data collection unit may be further configured to collect pressure values and inclination values from a pressure sensor and a gyro sensor attached to the chair.

In addition, the chair-sitting data collection unit may be further configured to synchronize the collected pressure values and inclination values with AI vision data implemented by a plurality of cameras.

In addition, a posture correction system according to another embodiment of the present invention for solving the technical problem may include the posture correction apparatus; and a user terminal communicably coupled with the posture correction apparatus.

### Advantageous Effects of Invention

According to the posture correction method and apparatus according to one embodiment of the present invention, users can receive feedback on the correction of their chair-sitting postures more quickly, thereby improving their quality of life.

In addition, according to the posture correction method and apparatus according to one embodiment of the present invention, it is possible to assist users who are in a wrong posture to correct their postures to right postures accurately by defining the right posture that the users should pursue and defining wrong postures similar to the right posture.

In addition, according to the posture correction method and apparatus according to one embodiment of the present invention, it is possible to analyze differences according to user's various chair-sitting postures using a gyro sensor to identify data on specific postures that cannot be identified with a pressure sensor alone, and thereby provide more user-customized posture correction information to the users.

In addition, according to the posture correction method and apparatus according to one embodiment of the present invention, it is possible to improve the accuracy of posture-specific analysis by confirming user's posture changes according to posture correction contents provided based on a constructed Al algorithm for posture correction and reflecting and updating the data of such posture changes back to the Al algorithm.

In addition, according to the posture correction method and apparatus according to one embodiment of the present invention, it is possible to further improve the accuracy of an AI posture analysis algorithm using an Al motion system based on a plurality of cameras to distinguish between right and wrong postures.

### Brief Description of Drawings

In order to fully understand the drawings cited in the detailed description of the present invention, a brief description of drawings is provided.
FIG. 1A illustrates a schematic diagram of a conventional posture correction chair, and FIG. 1B is an exemplary diagram explaining the limitations of the conventional posture correction chair.
FIG. 2 illustrates a schematic block diagram of a posture correction apparatus 100 according to one embodiment of the present invention and a posture correction system 10 including the same.
FIG. 3 is a flowchart explaining the overall flow of a posture correction method/algorithm implemented according to one embodiment of the present invention.
FIG. 4A is an exemplary diagram for explaining the seat structure of a chair 300 according to one embodiment of the present invention, and FIG. 4B is an exemplary diagram for explaining the backrest structure of the chair 300 according to one embodiment of the present invention.
FIG. 5 is an exemplary diagram for explaining a gyro sensor 330 and a pressure sensor 340 attached to the chair 300 according to one embodiment of the present invention.
FIG. 6A is an exemplary diagram for explaining the right and wrong postures defined when a user sits with their back against the backrest of a chair according to one embodiment of the present invention, and FIG. 6B is an exemplary diagram for explaining the right and wrong postures defining when a user does not sit with their back against the backrest of a chair according to one embodiment of the present invention.
FIG. 7 is a conceptual diagram for explaining Al vision data implemented by a plurality of cameras according to one embodiment of the present invention.
FIG. 8 illustrates an exemplary UI service screen of an Al posture correction application implemented for a user according to one embodiment of the present invention.

### Description of Embodiments

Hereinafter, embodiments according to the present invention will be described with reference to the attached drawings. When adding reference symbols to constituent elements in each drawing, it should be noted that the same constituent elements are given the same symbols as much as possible even if they are displayed in different drawings. In addition, when describing embodiments of the present invention, if it is determined that a specific description of a related known configuration or function interferes with the understanding of the embodiments of the present invention, a detailed description thereof will be omitted. In addition, although embodiments of the present invention will be described below, the technical idea of the present invention is not limited or restricted thereto and may be modified and implemented in various ways by those skilled in the art.

Throughout the specification, when a part is said to be "connected" to another part, this includes not only cases where it is "directly connected" but also cases where it is "indirectly connected" with another element in between. Throughout the specification, when a part is said to "include" a constituent element, this does not mean that other constituent elements are excluded, but rather that other constituent elements may be included, unless otherwise explicitly stated. In addition, terms such as first, second, A, B, (a), (b), and the like may be used to describe constituent elements of an embodiment of the present invention. These terms are only intended to distinguish the constituent elements from other constituent elements, and the nature, order, or sequence of the constituent elements is not limited by the terms.

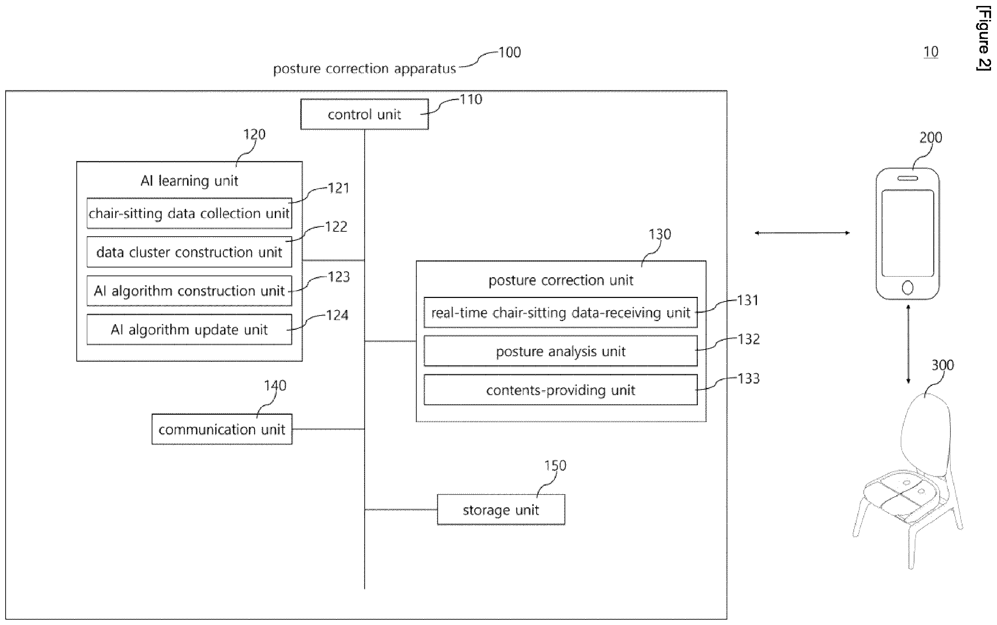

FIG. 2 illustrates a schematic block diagram of a posture correction apparatus 100 according to one embodiment of the present invention and a posture correction system 10 including the same.

As illustrated in the figure, the posture correction system 10 according to one embodiment of the present invention may include the posture correction apparatus 100, a user terminal 200, a chair 300, and the like. For reference, FIG. 2 illustrates a configuration in which a single user terminal 200 is communicably coupled with the posture correction apparatus 100 and the chair 300.However, this is merely an example for easy understanding of the present invention, and it is clear that a plurality of user terminals (for example, terminals of all family members such as a father, a mother, sisters, and brothers) can be communicably coupled with the posture correction apparatus 100 and the chair 300 under the condition of user identification.

The posture correction apparatus 100 according to one embodiment of the present invention may be configured to provide a service for correcting a user's chair-sitting posture in real-time to a user terminal 200 by collecting chair-sitting data for various postures using an ergonomically designed chair 300 and constructing and using an Al algorithm based on the data. In order to implement such a user-customized posture correction service, the posture correction apparatus 100 may include a control unit 110, an AI learning unit 120, a posture correction unit 130, a communication unit 140, a storage unit 150, and the like, as illustrated in FIG. 2.

For reference, the posture correction apparatus 100 according to one embodiment of the present invention corresponds to a device based on a self-computer program manufactured and operated by an individual or a private company. The posture correction apparatus 100 and may be referred to as various terms such as device, equipment, server, platform, and center according to various expression methods in the related technical field.

For example, the posture correction apparatus 100 according to one embodiment of the present invention may be configured to correct a user's chair-sitting posture in real-time by collecting chair-sitting data for various postures using an ergonomically designed chair 300 and constructing and using an Al algorithm based on the data. To this end, the posture correction apparatus 100 may be implemented to provide a user-customized posture correction service in the form of an application or a widget. Here a widget may refer to a mini-application that allows direct use of contents or functions without going through an application. Therefore, the term "application" in the present invention may be interpreted as a comprehensive concept including a widget, and a user may also download and install the posture correction apparatus 100 according to the present invention from an online market, for example, as a type of Al posture correction application.

For reference, the constituent elements 110, 120, 130, 140, and 150 of the posture correction apparatus 100 illustrated in FIG. 2 are merely exemplary constituent elements for explaining the operation, function, and the like of the posture correction apparatus 100 according to one embodiment of the present invention. Therefore, it is clear that the posture correction apparatus 100 according to one embodiment of the present invention may additionally include other constituent elements (for example, a power supply, a monitoring unit, a display unit, a notification unit, and the like) other than the illustrated constituent elements 110, 120, 130, 140, and 150.

The control unit 110 may be configured to comprehensively control various operations, functions, communications, and the like performed in the posture correction apparatus 100 according to one embodiment of the present invention. For example, the control unit 110 may be configured to comprehensively control the following operations: an operation of the Al learning unit 120 collecting and learning chair-sitting data, an operation of constructing a data cluster, an operation of collecting pressure and inclination values according to the user's sitting from the chair 300, an operation of synchronizing the collected pressure and inclination values with Al vision data implemented by a plurality of cameras, an operation of constructing an Al algorithm, an operation of updating the Al algorithm, an operation of the posture correction unit 130 receiving the user's chair-sitting data in real-time, an operation of analyzing the user's posture in real-time based on the Al algorithm, an operation of providing posture correction contents according to the analysis results, an operation of the communication unit 140 communicating with the user terminal 200, and an operation of the storage unit 150 storing various pieces of data.

For reference, the control unit 110 may be implemented as a processor, a controller, a microprocessor, a microcontroller, and the like, and may operate at least two or more of the constituent elements included in the posture correction apparatus 100 in combination to drive the application program.

The AI learning unit 120 collects and learns various pieces of chair-sitting data of users sitting on the chair 300 to construct a user-specific cluster, and constructs an Al algorithm for chair-sitting posture correction based on this. In this way, it is possible to provide a basis for the posture correction unit 130 to implement real-time analysis and correction of the user's postures.

For example, as exemplarily illustrated in FIG. 2, the Al learning unit 120 according to one embodiment of the present invention may include a chair-sitting data collection unit 121, a data cluster construction unit 122, an Al algorithm construction unit 123, and an Al algorithm update unit 124.

The chair-sitting data collection unit 121 may be configured to collect the chair-sitting data of a user on the chair 300.As one example, as one of the data required to construct an Al algorithm for posture correction, chair-sitting data for a plurality of users (for example, thousands to tens of thousands of users) may be collected. Here, when each user adjusts the backrest and seat provided on the chair 300 to fit their size and sits on the chair 300, user information is additionally input for data learning, and the pressure/gyro sensor operation is ready after a predetermined calibration, the chair-sitting data collection unit 121 can collect the chair-sitting data of each user.

Here, the collected user information may include nationality (for example, body type, 15 detailed upper/lower body lengths, and the like), disease (for example, spinal disease/adult disease, posture according to symptom severity, and the like), age (for example, under 50, over 50), occupation (for example, daily sitting time, work characteristics, and the like), lifestyle pattern (for example, usual posture, activity pattern, and the like), gender (for example, male/female, gender-specific habitual posture, and the like), but this information is only an example of user information and is not limited thereto.

In addition, detailed body shape information of the user may be additionally used when constructing an Al algorithm for posture correction. For example, detailed body shape information may be used to compare and analyze specific parts of the user's upper and lower body. Such detailed user body shape information may include, but is not limited to, information such as neck-hip dimensions, shoulder-hip dimensions, inner arm dimensions, total arm dimensions, head-to-sole dimensions, and shoulder-to-sole dimensions.

In addition, a plurality of users may sit on the chair 300 according to one embodiment of the present invention, and chair-sitting data may be collected from each of the plurality of users. To this end, the user terminal 200 synchronized with short-range wireless communication, for example, Bluetooth, may be identified, and then chair-sitting data may be collected based on the information of the identified user. For example, when a user sitting on the chair 300 changes from a father of 180 cm/80 kg to a daughter of 150 cm/30 kg, the chair-sitting data collection unit 121 according to one embodiment of the present invention can automatically change its user mode to collect chair-sitting data such as pressure values and gyro values based on the input user information of the daughter.

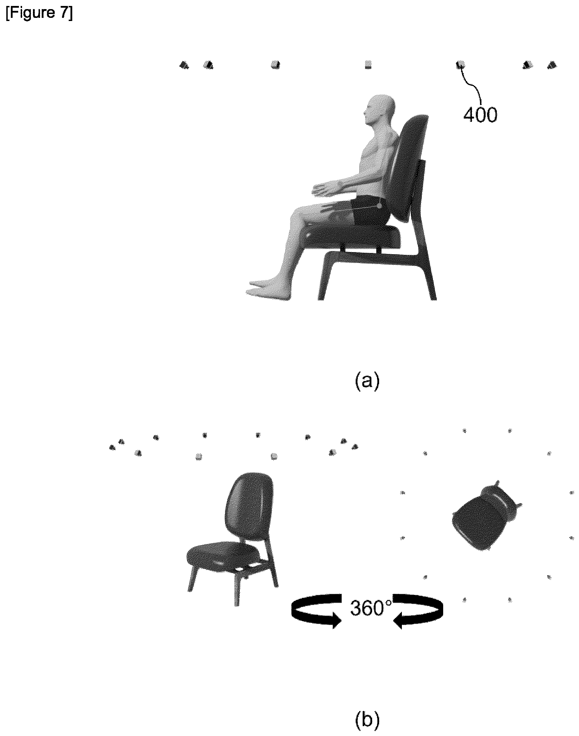

For example, the chair-sitting data collection unit 121 according to one embodiment of the present invention can be additionally configured to collect pressure values and inclination values from a pressure sensor and a gyro sensor attached to the chair 300, and synchronize the pressure values and inclination values thus collected with Al vision data implemented by a plurality of cameras. The chair-sitting data collection unit according to one embodiment of the present invention is characterized by implementing posture correction of users using AI vision data, and such AI vision data will be described in more detail in FIG. 7 below.

The data cluster construction unit 122 may be configured to construct a user-specific data cluster based on the collected chair-sitting data. More specifically, the data cluster construction unit 122 can construct a data cluster among users of similar types based on the unique data of each user. Here, the data cluster construction unit 122 can analyze the reason for taking a specific posture (for example, crossing the legs or sticking out the buttocks, and the like) using AI modeling (for example, posture changes due to habits or occupation, or specific postures due to diseases or the like) and analyze the user's posture pattern from such common data.

The Al algorithm construction unit 123 may be configured to construct an Al algorithm for posture correction based on the constructed data cluster. The constructed Al algorithm can function as a program, software, and the like for correcting the user's posture, and for example, can be the basis for providing a user-customized posture correction service to the user terminal 200 in the form of an application such as a so-called "Al posture correction application". In addition, the constructed Al algorithm can be the basis for the posture correction unit 130 to analyze the user's chair-sitting posture, correct the posture, and provide contents related to posture correction.

When an Al algorithm for correcting the user's posture is constructed by the Al algorithm construction unit 123, the Al algorithm update unit 124 may be configured to analyze the user's improvement items using the constructed Al algorithm. For example, the Al algorithm update unit 124 can analyze and identify the improvement items that the user should take using Al. More specifically, the Al algorithm update unit 124 can identify the correlation between posture and pain by identifying the time the user sits on the chair 300 and the frequency of correct/wrong postures and prepare necessary information for the user accordingly.

In addition, the Al algorithm update unit 124 may be additionally configured to update the Al algorithm by applying all of the user's posture improvement situation, additional consideration items, and the like (for example, changes in body weight, changes in height, changes in posture, and the like) using machine learning technology or the like. Important information according to changes in user information along with the update of the Al algorithm may be constructed as Big-Data and stored in the storage unit 150 and the like, so that it can be used by medical institutions and the like in the future.

The posture correction unit 130 may be configured to provide a user-customized posture correction service according to the results of learning various pieces of user's chair-sitting data based on the Al algorithm in the Al learning unit 120.In order to implement such operation and service, the posture correction unit 130 according to one embodiment of the present invention may include a real-time chair-sitting data-receiving unit 131, a posture analysis unit 132, and a contents-providing unit 133.

The real-time chair-sitting data-receiving unit 131 may be configured to receive real-time chair-sitting data displayed according to the user currently sitting on the chair 300 from the user terminal 200, more specifically, from the user terminal 200 in which the Al posture correction application is downloaded and installed and which is wirelessly communicably connected to the chair 300.When installing and downloading an AI posture correction application, a user may additionally input predetermined user information (for example, nationality, disease, age, occupation, lifestyle pattern, gender, and the like), and the user information thus input may be additionally used when the posture analysis unit 132 analyzes the user's chair-sitting posture.

More specifically, the posture analysis unit 132 may be configured to analyze the user's posture in real-time and provide information on posture correction based on the Al algorithm constructed and updated in the Al learning unit 120 and the real-time chair-sitting data received from the user terminal 200.

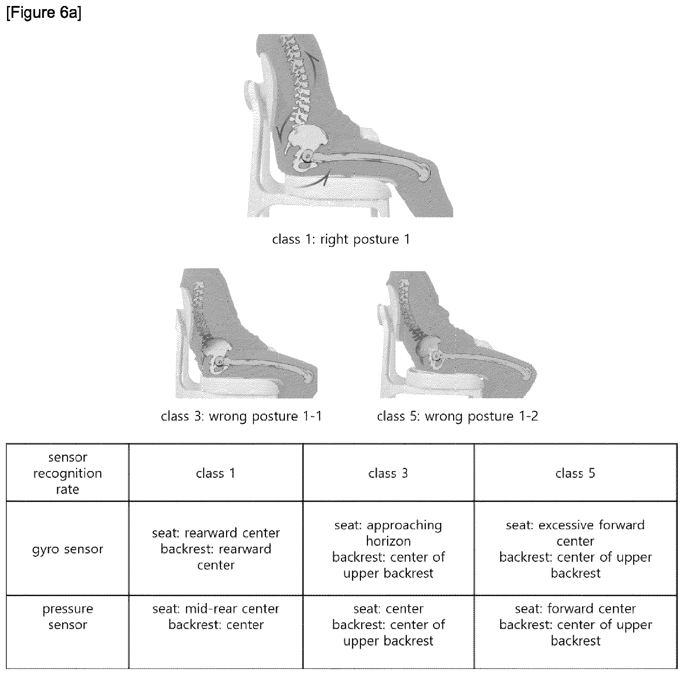

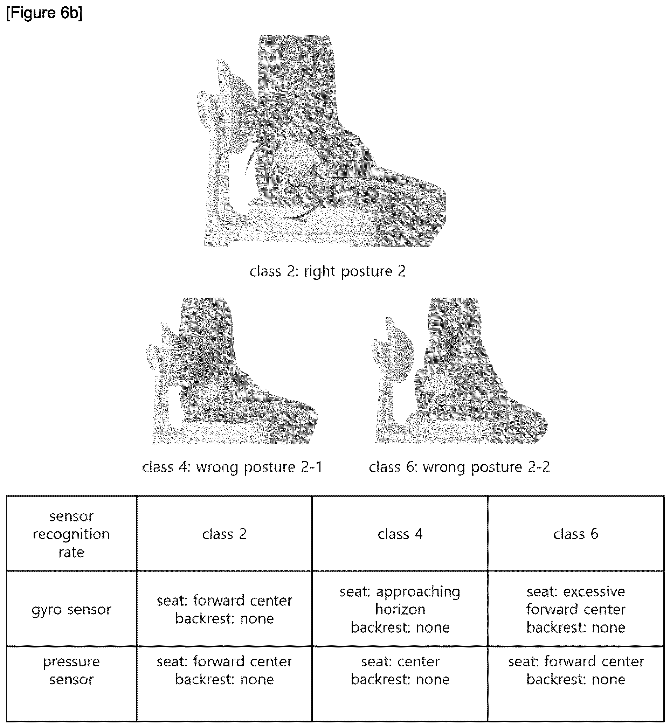

For example, as described in FIG. 6 below, at least one right posture (that is, a correct posture) and at least one wrong posture (that is, a bad posture) may be defined as criteria for implementing a posture correction service according to an embodiment of the present invention. The posture analysis unit 132 may determine whether the user's current posture is a right posture or a wrong posture, and may provide posture correction information to a user who is in a wrong posture so that the user can correct the wrong posture and return to the right posture. Since the determination of whether the posture is a right/wrong posture is based on an Al algorithm learned on a data cluster constructed based on user information, a more user-customized posture correction service may be provided.

For example, correction of the user's chair-sitting posture analyzed by the posture analysis unit 132 can be provided to the user terminal 200 in real-time. Here, the user terminal 200 can additionally be configured with an interface for selecting a mode for posture correction, and for example, the mode for posture correction may include a normal mode and a training mode.

More specifically, the normal mode may correspond to a mode in which, when a user sits on the chair 300 for a predetermined period, the analysis results are provided as scores, statistics, and the like, or a guide is provided after the period ends. In addition, the training mode is a mode provided to users who want to more actively correct their postures, and may correspond to a mode implemented so that a training time such as "10 minutes" or "20 minutes" is set and feedback on posture correction is provided immediately during the set training time.

The contents-providing unit 133 may be configured to provide the user with various contents related to posture and health based on data related to the analysis of the user's postures analyzed by the posture analysis unit 132.For example, the contents-providing unit 133 may provide the user with various contents related to posture or health, such as contents related to diseases that may be caused by a specific posture, contents related to the prevention of a specific disease, contents related to the management of a specific disease, and contents that can share medical knowledge. By providing the user with additional contents related to posture, health, diseases, and the like, in addition to information related to posture correction, the user's satisfaction with the Al posture correction service may be further improved.

The communication unit 140 may be configured to communicate with the user terminal 200.For example, when the user sits on the chair 300 at home, data such as pressure values, gyro values, and the like detected by sensors attached to the chair 300 may be received from the user terminal 200 through the communication unit 140.In addition, an Al algorithm learned and evolved based on user-specific data clusters, information on user's sitting and correction analyzed based on user's chair-sitting data received in real-time, and various contents related thereto may be transmitted to the user terminal 200 through the communication unit 140.

For reference, the communication unit 140 is provided for direct connection with the outside or connection through a network, and may be implemented as a wired and/or wireless communication unit 140.More specifically, the communication unit 140 may transmit data from the control unit 110, AI learning unit 120, posture correction unit 130, storage unit 150, and the like, through wires or wirelessly, and receive data from the outside, through wires or wirelessly, transmit the data to the control unit 110, Al learning unit 120, and posture correction unit 130, and store the data in the storage unit 150.The data may include contents such as text, images, and moving images.

The communication unit 140 may communicate through LAN, WCDMA (Wideband Code Division Multiple Access), LTE (Long Term Evolution), WiBro (Wireless Broadband Internet), RF (Radio Frequency) communication, Wireless LAN, Wi-Fi (Wireless Fidelity), NFC (Near Field Communication), Bluetooth, infrared communication, and the like. However, this is exemplary, and various wired and wireless communication technologies applicable in the relevant technical field can be used depending on the embodiment to which the present invention is applied.

The storage unit 150 may be configured to store any data, materials, contents, records, and the like related to various operations, functions, and the like of the posture correction apparatus 100 according to one embodiment of the present invention. For example, the storage unit 150 may store user information, user cluster information, information on Al algorithms, information on defined right/wrong postures, information on user's improvement items, information on big data, information on posture/health-related contents, and the like.

For reference, the storage unit 150 may be implemented as various types of storage devices capable of inputting and outputting information, such as HDD (Hard Disk Drive), ROM (Read Only Memory), RAM (Random Access Memory), EEPROM (Electrically Erasable and Programmable Read Only Memory), flash memory, CF (Compact Flash) card, SD (Secure Digital) card, SM (Smart Media) card, MMC (Multimedia) card, or Memory Stick, as known to those skilled in the art. The storage unit 150 may be provided inside the posture correction apparatus 100 or provided in a separate external apparatus, as illustrated in FIG. 2. Alternatively, the storage unit 150 may be replaced with a web storage that performs a storage function on the Internet.

The user terminal 200 may be configured to be wirelessly communicably connected to the posture correction apparatus 100 and the chair 300 by downloading and installing a predetermined application, for example, an Al posture correction application. Although FIG. 2 exemplary illustrates a single user terminal 200, as described above, a plurality of user terminals 200 may be communicably coupled with the posture correction apparatus 100 and the chair 300 based on user identification. Therefore, as long as there is the chair 300 configured with a pressure sensor and a gyro sensor attached to the seat and backrest, there is an advantage in that any family member or any company member can receive a user-customized posture correction service according to the present invention.

For reference, the user terminal 200 may also be referred to as a subscriber unit, a subscriber station, a mobile station, a mobile terminal, a remote station, a remote terminal, a mobile device, an access terminal, a terminal, a wireless communication device, a user agent, a user device, or user equipment (UE).The access terminal may be a cellular telephone, a cordless telephone, a session initiation protocol (SIP) telephone, a wireless local loop (WLL) station, a personal digital assistant (PDA), a handheld device with wireless access capability, a computing device, or another processing device accessible to a wireless modem.

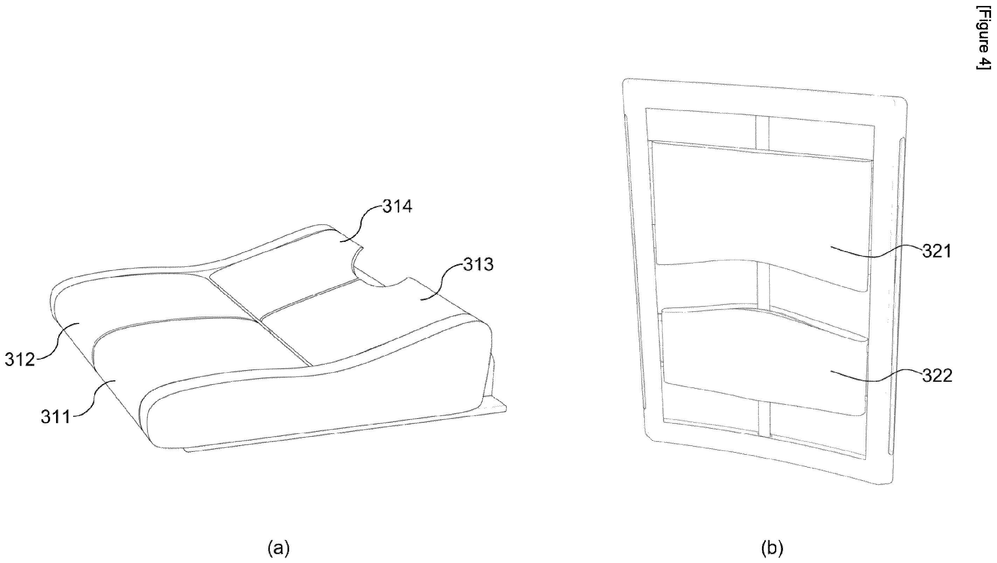

The chair 300 is a means for receiving a posture correction service in real-time while the user is sitting thereon, and may be implemented so that the four seats can move independently forward-backward-left-right and the two backrests can also move independently up-down-left-right. For reference, the chair 300 used in the posture correction system 10 according to one embodiment of the present invention is based on the structural mechanism of Korean Patent No. 10-2136560 (chair seat and chair including the same) held by the applicant as a patentee and additionally implements the attachment of sensors for correcting the user's posture. The configuration of this chair 300 will be described in more detail in FIG. 4 below.

For reference, the name "chair 300" is used in the following specification. However, the chair 300 according to one embodiment of the present invention is may be referred to as an "loT chair" because it is an Internet-of-Things-based chair 300 configured to transmit data obtained from the attached pressure sensor and/or gyro sensor to the user terminal 200.Alternatively, the chair 300 may be referred to as another term such as a "healthcare chair" in that it is implemented for the ultimate purpose of managing the user's health through the chair 300.But the term "chair 300" will be used below to collectively refer to these various expressions regarding chairs.

With the user-customized posture correction service implemented and provided by the posture correction apparatus 100 illustrated in FIG. 2 and the posture correction system 10 including the same, the users can receive feedback on the correction of their chair-sitting postures more quickly. Moreover, it is possible to assist users who are in a wrong posture to correct their postures to right postures accurately by defining the right posture that the users should pursue and defining wrong postures similar to the right posture. Furthermore, it is possible to analyze differences according to user's various chair-sitting postures using a gyro sensor to identify data on specific postures that cannot be identified with a pressure sensor alone. Furthermore, it is possible to improve the accuracy of posture-specific analysis by confirming user's posture changes according to posture correction contents provided based on a constructed Al algorithm for posture correction and reflecting and updating the data of such posture changes back to the Al algorithm. Furthermore, it is possible to further improve the accuracy of an AI posture analysis algorithm using an Al motion system based on a plurality of cameras to distinguish between right and wrong postures.

Hereinafter, there will be a more detailed description of the overall flow of the user-customized posture correction service implemented by the posture correction apparatus 100 and the posture correction system 10 including the posture correction apparatus 100, the structure of the chair 300, the definitions of right and wrong postures, AI vision data, service UI, and the like.

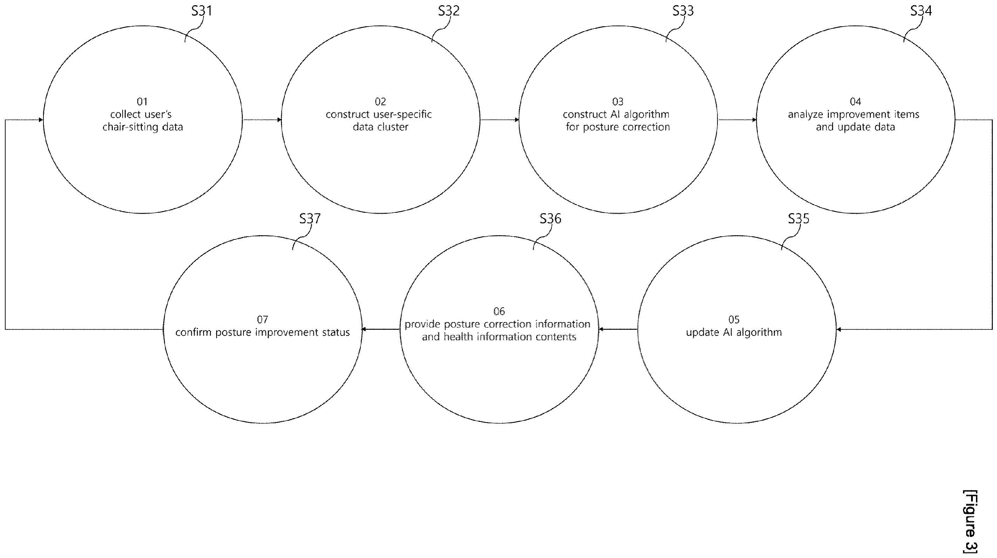

FIG. 3 is a flowchart for explaining the overall flow of the posture correction method/algorithm implemented according to one embodiment of the present invention.

First, the chair-sitting data collection unit 121 of the Al learning unit 120 can collect the user's chair-sitting data (S31). More specifically, the chair-sitting data collection unit 121 may be configured to collect chair-sitting data for a plurality of users, for example, thousands to tens of thousands of users. Here, each user adjusts the backrest and seat provided on the chair 300 to fit their size and sits on the chair 300, and user information such as nationality, disease, age, occupation, lifestyle pattern, gender, and the like, may be additionally input for data learning. After the pressure/gyro sensor operation is ready through a predetermined correction or calibration, the chair-sitting data of each user may be collected.

Here, individual chair-sitting data may be collected for each of a plurality of users sitting on the chair 300.For example, the user terminal 200 synchronized with Bluetooth (BL) may be identified and the chair-sitting data according to a user classification or a user group may be collected based on the identified user information.

When the chair-sitting data for a plurality of users is collected, the data cluster construction unit 122 of the AI learning unit 120 can construct a user-specific data cluster based on the collected chair-sitting data (S32). More specifically, the data cluster construction unit 122 can construct a data cluster for users with similar types (or classifications) based on the unique data possessed by each user. Here, the data cluster construction unit 122 can analyze the reason why the user takes a specific posture such as crossing the legs or sticking out the buttocks using the Al modeling and analyze the user's posture pattern from such common data.

When the chair-sitting data for a plurality of users is continuously collected and a user-specific data cluster is constructed, the Al algorithm construction unit 123 of the Al learning unit 120 can construct an Al algorithm for correcting the user's posture more rapidly and accurately (S33).

For example, the Al algorithm constructed by the Al algorithm construction unit 123 can function as a program, software, and the like for correcting the user's posture, and for example, can be the basis for providing a user-customized posture correction service to the user terminal 200 in the form of an Al posture correction application. In addition, the constructed Al algorithm can be the basis for the posture correction unit 130 to analyze the user's chair-sitting posture, correct the posture, and provide contents related to posture correction.

When an Al algorithm is constructed by the Al algorithm construction unit 123, the Al algorithm update unit 124 of the Al learning unit 120 can analyze the user's improvement items using the constructed Al algorithm and update the related data and Al algorithm based on the analyzed improvement items (S34, S35).

For example, the Al algorithm update unit 124 can analyze and identify the improvement items that the user should take using Al. More specifically, the Al algorithm update unit 124 can identify the correlation between posture and pain by identifying the time the user sits on the chair 300 and the frequency of correct/wrong postures and prepare necessary information for the user accordingly.

As described above, the Al algorithm update unit 124 may be additionally configured to update the Al algorithm by applying all of the user's posture improvement situation, additional consideration items, and the like using machine learning technology or the like. Important information according to changes in user information along with the update of the Al algorithm may be constructed as big data and stored in the storage unit 150 and the like, so that it can be used by medical institutions and the like in the future.

In this way, when the AI learning unit 120 performs learning of user-specific chair-sitting data, construction of a data cluster, analysis of improvement items, and update of data and Al algorithms, the posture correction unit 130 can provide a user-customized posture correction service according to the results of learning various pieces of user's chair-sitting data based on the updated Al algorithm and provide various health/posture-related contents to the user terminal 200 (S36).

When the user-customized posture correction service and various health/posture-related contents are provided to users, the users can check how much change has occurred over a set period, that is, the status of posture improvement (S37).By collecting and using the changed posture information as chair-sitting data (S31), it is possible to increase the accuracy of posture analysis can be increased, and ultimately, assist improvement in the user's posture by repeatedly going through the user-customized posture correction service.

Through such a user-customized posture correction service, Al algorithms can identify the characteristics of each posture that are difficult for people to recognize, and more accurate posture correction can be implemented and provided. When users change their postures with the right balance, pain and discomfort caused by wrong postures can be reduced, thereby minimizing unnecessary costs and time.

In addition, posture correction can help prevent secondary diseases such as cervical spine deformity, hypertension, and myocardial infarction, and it can perform the function of anatomically and ergonomically restoring posture habits for modern people who are naturally more likely to be exposed to wrong postures as the time spent using electronic devices such as cell phones and laptops increases.

FIG. 4A is an exemplary diagram for explaining the seat structure of the chair 300 according to one embodiment of the present invention, and FIG. 4B is an exemplary diagram for explaining the backrest structure of the chair 300 according to one embodiment of the present invention.

As described above, the chair 300 used in the learning of an Al algorithm according to one embodiment of the present invention and implementing and providing a user-customized posture correction service according to the learning algorithm can use the configuration of an ergonomically designed chair described in Korean Patent No. 10-2136560 held by the applicant of the present invention.

FIG. 4A is an exemplary diagram for explaining the seat structure of the chair 300 according to one embodiment of the present invention that is ergonomically designed. More specifically, the seat of the chair 300 according to one embodiment of the present invention may be implemented with four seat units 311, 312, 313, and 314 configured with a plurality of frames that are independently configured and implemented to be movable, a support unit configured to support the frames while being coupled with the frames at the lower side of the plurality of frames, and memory foam covering the plurality of frames. For example, the independent movement of the plurality of frames can be implemented by combining the omnidirectional movement of the center ball within the recess and the flexible movement of the plurality of frames with respect to a spring group, which shows a significant difference from a conventional fixedly mounted seat of an ordinary chair.

For example, the seat units 311, 312, 313, and 314 of the chair 300 according to one embodiment of the present invention are independently movable so that the user's chin is lowered vertically to look straight ahead and the cervical spine naturally forms a C-curve.

In addition, FIG. 4B exemplarily illustrates a backrest configuration of the chair 300 according to one embodiment of the present invention that is ergonomically designed. More specifically, the backrest of the chair 300 according to one embodiment of the present invention is provided with a plurality of (for example, two) backrest frames, each of which is implemented in a hollow shape and is implemented to enable independent movement, and these two backrest frames are implemented to be able to move up and down or left and right.

By covering these backrest frames with backrest memory foam, two backrest units 321 and 322 can be implemented. Like the seat of the chair 300, the backrest of the chair 300 can also enable flexible movement of a plurality of frames and independent movement accordingly.

Also, although not explicitly illustrated in the drawing, an operating means, for example, a stepping motor, can be operated according to the length of the user's upper/lower body to store the position. Accordingly, the most accurate and comfortable posture correction can be implemented according to the user's body type. For example, with the Al algorithm-based user-customized posture correction service according to the present invention, turtle neck can be prevented by stabilizing the splenius cervicis and splenius capitis muscles, and cervical spine curvature can be prevented by stabilizing the serratus posterior superior and serratus posterior inferior. In addition, the limitations in body type such as weight and height can be overcome, a plurality of users can use it based on user identification, and anyone can be guided to have the correct posture regardless of body type, gender, and the like.

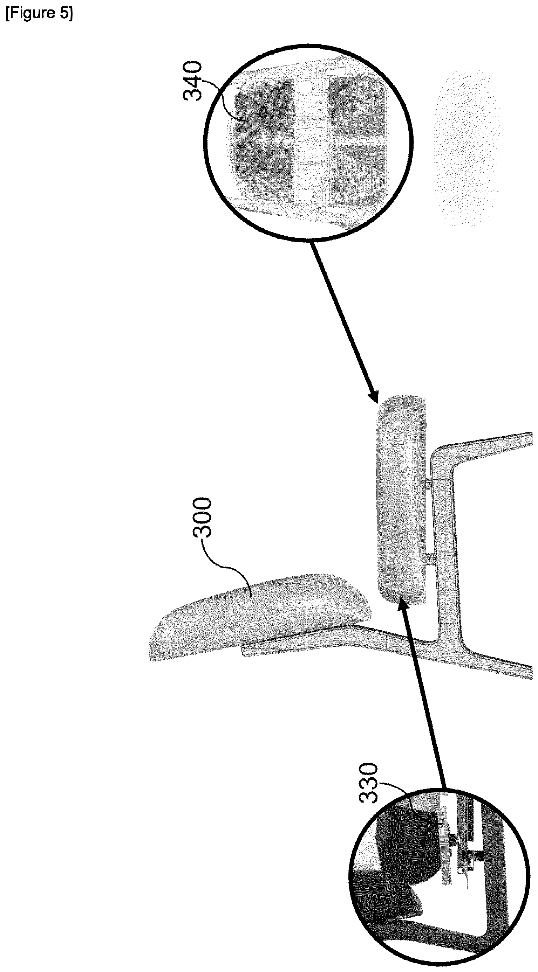

As illustrated in FIG. 4, the seat and the backrest of the chair 300 according to one embodiment of the present invention are provided, and predetermined sensors may be additionally provided in the chair 300 according to one embodiment of the present invention for a process such as constructing an Al algorithm and analyzing user's various postures to provide a user-customized posture correction service. An example of such a sensor arrangement is illustrated in FIG. 5.

FIG. 5 is an exemplary diagram for explaining a gyro sensor 330 and a pressure sensor 340 attached to the chair 300 according to an embodiment of the present invention.

As exemplarily illustrated in FIG. 5, the chair 300 according to one embodiment of the present invention may be equipped with the gyro sensor 330 and the pressure sensor 340.Thus, it can be clearly contrasted with existing posture correction chairs that only have the pressure sensor 340 that can only receive data vertically and thus the accuracy of sensing is greatly reduced.

More specifically, in order to obtain data on the user's posture that does not act vertically on the pressure sensor 340, such as inclination data, the gyro sensor 330 may be additionally attached to the chair 300, more specifically, to each of the seat units 311, 312, 313, and 314 of the seat and each of the backrest units 321 and 322 of the backrest, in addition to the pressure sensor 340.In this way, more accurate pressure values and inclination values can be detected for various postures that the user can take with respect to the chair 300.

For example, the gyro sensor 330 can analyze the angle or inclination to obtain data that is difficult to detect by the pressure sensor 340 because it does not act vertically to the pressure sensor 340 depending on the user's posture. The pressure sensor 340 can sense the distribution of pressure depending on the user's posture. The posture analysis unit 132 of the posture correction unit 130 can analyze the user's posture more precisely and accurately (for example, analyze the difference depending on the user's posture) using the pressure value from the pressure sensor 340 and the inclination value (or angle value) from the gyro sensor 330.

In addition, since existing posture correction chairs are not ergonomically designed and are usually configured by attaching a sensor pad inside a cushion, there are problems such as data loss due to detection data being transmitted when the film is crumpled or rolled up. However, the pressure sensors 340 according to one embodiment of the present invention are individually and independently attached to the four seat units 311, 312, 313, and 314 and the two backrest units 321 and 322, so that data loss of the pressure sensor 340 can be minimized.

For reference, the pressure sensors 340 may be attached to the seat units 311, 312, 313, and 314 and the backrest units 321 and 322 as individual sensors, Alternatively, the pressure sensors 340 may be attached in the form of a pad to the surfaces of the seat units 311, 312, 313, and 314 and the backrest units 321 and 322 to implement more improved sensing accuracy.

FIG. 6A is an exemplary diagram explaining the right and wrong postures defined when a user sits with their back against the backrest of a chair according to one embodiment of the present invention, and FIG. 6B is an exemplary diagram explaining the right and wrong postures defined when a user does not sit with their back against the backrest of a chair according to one embodiment of the present invention.

As described above, in order to implement a user-customized posture correction service according to one embodiment of the present invention, various right and wrong postures can be defined in advance when constructing an Al algorithm, and examples of such right and wrong postures are illustrated in FIGS. 6A and 6B, respectively. For reference, in the present invention, "right posture" can be defined as a posture in which the body's balance is properly maintained so that joints, nerves, and muscles are not strained when the user is sitting on the chair 300.

For example, FIG. 6A illustrates an exemplary diagram for explaining right and wrong postures defined when a user sits with their back against the backrest of a chair according to one embodiment of the present invention. The right posture 1 can be defined as class 1 as a desirable posture that a user should take when leaning their back against the backrest of a chair. For example, this right posture 1 can be defined such that the gyro sensor 330 detects that both the seat and the backrest face backward center, and the pressure sensor 340 detects that the seat faces the mid-rear center and the backrest faces the center.

In addition, when a user leans their back against the backrest of a chair, many users mistakenly believe that this is the right posture. However, in reality, it is a wrong posture that puts a strain on the lower back, and wrong posture 1-1 can be defined as class 3.For example, this wrong posture 1-1 can be defined such that the gyro sensor 330 determines that the seat approaches the horizon and the backrest faces the center of the upper backrest and the pressure sensor 340 determines that the seat faces the center and the backrest faces the center of the upper backrest.

In addition, the wrong posture 1-2 that should not be taken when the user leans back against the backrest of the chair can be defined as class 5.For example, the wrong posture 1-2 can be defined such that the gyro sensor 330 determines that the seat faces the excessive forward center and the backrest faces the center of the upper backrest and the pressure sensor 340 determines that the seat faces the forward center and the backrest faces the center of the upper backrest.

Similarly, FIG. 6B illustrates an exemplary diagram for explaining the right and wrong postures defined when the user does not lean back against the backrest of the chair according to one embodiment of the present invention. The right posture 2 can be defined as class 2 as a desirable posture to be taken when the user does not lean their back against the backrest of the chair. For example, this right posture 2 can be defined such that the gyro sensor 330 determines that the seat faces the forward center and the pressure sensor 340 determines that the seat faces the forward center.

In addition, when the user does not lean their back against the backrest of the chair, many users mistakenly believe that this is the right posture. However, in reality, it is a wrong posture that puts a strain on the lower back, and wrong posture 2-1 can be defined as class 4. For example, this wrong posture 2-1 can be defined such that the gyro sensor 330 determines that the seat approaches the horizon and the pressure sensor 340 determines that the seat faces the center.

In addition, the wrong posture 2-2 that should not be taken when the user does not lean their back against the backrest of the chair can be defined as class 6. For example, this wrong posture 2-2 can be defined such that the gyro sensor 330 determines that the seat faces the excessive forward center and the pressure sensor 340 determines that the seat faces the forward center.

For reference, in the example of FIG. 6, the inclination value from the gyro sensor 330 is described as the direction or distribution, and the pressure value from the pressure sensor 340 is also described as the direction or distribution for a common and universal expression. However, in implementing a user-customized posture correction service according to one embodiment of the present invention, the range of the inclination values from the gyro sensor 330 defined for each user cluster and the range of the pressure values from the pressure sensor 340 defined for each user cluster can be derived, and analysis and determination of the right and wrong postures for each class can be preferably performed based on these inclination values and/or pressure values.

In addition, in FIG. 6, one right posture and two wrong postures, a total of six postures (two right postures and four wrong postures), are described for each posture, that is, when the user leans against the backrest and when the user does not lean against the backrest. However, this is only an example for easy understanding of the present invention, and it will be obvious that the number of right and wrong postures defined in advance for correcting the user's posture may vary depending on various implementation examples or various embodiments. More preferably, the accuracy of posture analysis may be improved by increasing the number.

For example, the detection values (inclination values and pressure values) defined as right or wrong postures may vary depending on user classification or grouping, and a more optimized and more accurate user-customized posture correction service may be provided to each user through such user classification.

FIG. 7 is a conceptual diagram for explaining Al vision data implemented by a plurality of cameras according to one embodiment of the present invention.

According to one embodiment of the present invention, the pressure values collected by the pressure sensor 340 attached to the chair 300 and the inclination values collected by the gyro sensor 330 attached to the chair 300 are stored in a synchronized manner with Al vision data implemented by a plurality of cameras 400, thereby enabling more accurate and precise analysis of user's various postures. An example of an AI vision system implemented by the plurality of cameras 400 is exemplarily illustrated in FIG. 7.

As exemplarily illustrated in FIG. 7, using an Al motion system, the user's right and wrong postures can be distinguished more accurately. For example, the accuracy can be significantly improved when implementing the data of user's six postures (that is, two right postures and four wrong postures) visualized through vision as sensor data expressed in numbers. For example, computer vision can be measured with a plurality of cameras 400, for example, up to 12 cameras 400, skeletons values, joint point values, and the like can be generated for each user's posture (for example, based on HPE (Human Pose Estimation) technology), and the user's sitting can be photographed from all directions of 360 degrees by up to 12 cameras 400, so that the inclination values and pressure values obtained for each of the user's various postures can be accurately synchronized.

That is, by analyzing the posture according to the user's posture distribution, analyzing the body data of each user, analyzing the motion data according to the real-time posture change, and analyzing the specific seat and backrest angles for each posture, the accuracy of the AI posture analysis algorithm through the combination of real-time vision data and sensor integration data can be significantly improved.

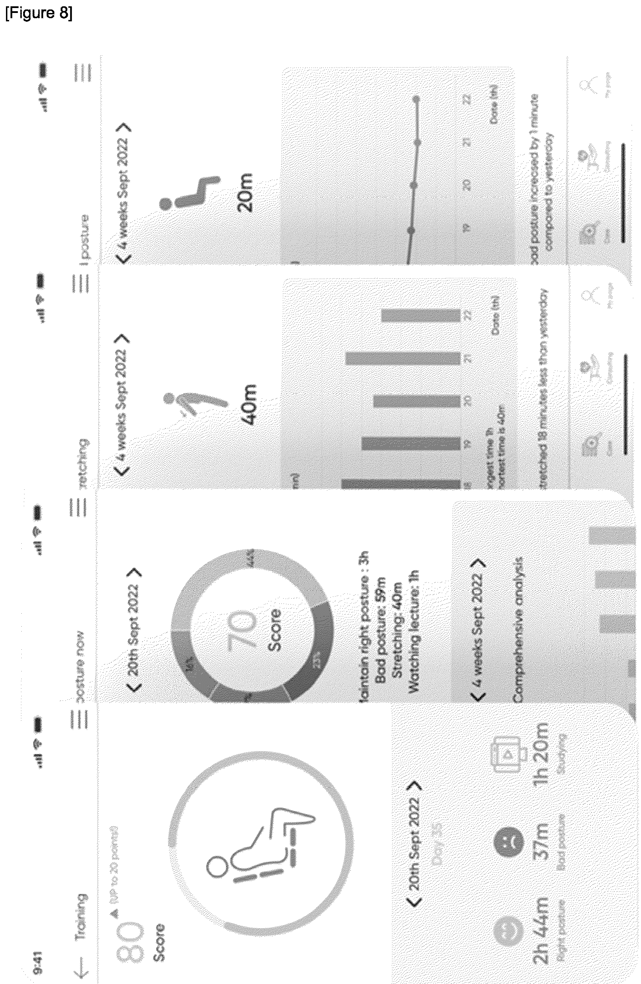

FIG. 8 illustrates an exemplary UI service screen of an Al posture correction application implemented for a user according to one embodiment of the present invention.

As a type of so-called Al posture correction application, a user-customized posture correction service according to one embodiment of the present invention can be provided to a user through the user terminal 200, and an example of such a user interface (UI) is illustrated in FIG. 8.As illustrated, each user can receive a score for a correct posture, various analysis results, advice for posture improvement, statistics, and the like through the user-customized posture correction service provided through the Al posture correction application.

For example, the user's real-time posture analysis can provide relevant right posture information so that the user can maintain the right posture, and the training mode can provide more intensive and fast-feedback correction training for a preset period. Furthermore, it is possible to provide daily, weekly, and monthly comparison results and growth rates of right and wrong postures and provide customized medical information, health contents, and the like to the user terminal 200.

As an example, a user with excellent posture correction effects can be provided with a certain reward (for example, points, savings, and the like), and thus, user's interest and participation in the user-customized posture correction service can be more actively induced.

As described above, according to the posture correction method and apparatus according to one embodiment of the present invention, users can receive feedback on the correction of their chair-sitting postures more quickly, thereby improving their quality of life.

In addition, according to the posture correction method and apparatus according to one embodiment of the present invention, it is possible to assist users who are in a wrong posture to correct their postures to right postures accurately by defining the right posture that the users should pursue and defining wrong postures similar to the right posture.

In addition, according to the posture correction method and apparatus according to one embodiment of the present invention, it is possible to analyze differences according to user's various chair-sitting postures using a gyro sensor to identify data on specific postures that cannot be identified with a pressure sensor alone, and thereby provide more user-customized posture correction information to the users.

In addition, according to the posture correction method and apparatus according to one embodiment of the present invention, it is possible to improve the accuracy of posture-specific analysis by confirming user's posture changes according to posture correction contents provided based on a constructed Al algorithm for posture correction and reflecting and updating the data of such posture changes back to the Al algorithm.

In addition, according to the posture correction method and apparatus according to one embodiment of the present invention, it is possible to further improve the accuracy of an AI posture analysis algorithm using an Al motion system based on a plurality of cameras to distinguish between right and wrong postures.

Meanwhile, various embodiments described in this specification can be implemented by hardware, middleware, microcode, software, and/or a combination thereof. For example, various embodiments can be implemented in one or more application-specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, other electronic units designed to perform the functions presented herein, or a combination thereof.

In addition, for example, various embodiments can be contained or encoded in a computer-readable medium containing instructions. Instructions contained or encoded in a computer-readable medium can cause a programmable processor or other processor to perform a method, for example, when the instructions are executed. The computer-readable medium includes a computer storage medium, which may be any available medium that can be accessed by a computer. For example, such a computer-readable medium may include RAM, ROM, EEPROM, CD-ROM or other optical disc storage media, magnetic disk storage media, or other magnetic storage devices.

This hardware, software, firmware, and the like may be implemented within the same device or separate devices to support the various operations and functions described herein. Additionally, constituent elements, units, modules, components, and the like described as "xxx units" in the present invention may be implemented together or individually as separate but interoperable logic devices. The depiction of different features for modules, units, and the like is intended to emphasize different functional embodiments and does not necessarily imply that they must be realized by separate hardware or software components. Rather, the functions associated with one or more modules or units may be performed by separate hardware or software components or integrated into common or separate hardware or software components.

While operations are depicted in the drawings in a particular order, it should not be understood that these operations must be performed in the particular order illustrated or in a sequential order, or that all the illustrated operations must be performed to achieve desired results. In some environments, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

As described above, the best embodiment has been disclosed in the drawings and the specification. Although specific terms have been used herein, they have been used only for the purpose of describing the present invention and have not been used to limit the meaning or the scope of the present invention described in the claims. Therefore, those skilled in the art will understand that various modifications and equivalent other embodiments are possible from this. Therefore, the true technical protection scope of the present invention should be determined by the technical idea of the appended claims.

### Reference Signs List

100: Posture correction apparatus
110: Control unit
120: Al learning unit
121: Chair-sitting data collection unit
122: Data cluster construction unit
123: Al algorithm construction unit
124: Al algorithm update unit
130: Posture correction unit
131: Real-time chair-sitting data-receiving unit
132: Posture analysis unit
133: Contents-providing unit
140: Communication unit
150: Storage unit
200: User terminal
300: Chair
311, 312, 313, 314: Seat unit
321, 322: Backrest unit
330: Gyro sensor
340: Pressure sensor
400: Camera
10: Posture correction system

## Claims

1. A posture correction method, comprising:
collecting chair-sitting data of users on a chair;
constructing a user-specific data cluster based on the collected chair-sitting data;
constructing an Al algorithm for posture correction based on the constructed data cluster; and
providing information on posture correction based on the constructed Al algorithm and real-time chair-sitting data.

2. The posture correction method according to claim 1, further comprising:
analyzing improvement items of the users using the constructed Al algorithm; and
updating the Al algorithm based on the analyzed improvement items.

3. The posture correction method according to claim 1, wherein
the collecting of the chair-sitting data of the users on the chair includes collecting pressure values and inclination values from a pressure sensor and a gyro sensor attached to the chair.

4. The posture correction method according to claim 3, wherein
the collecting of the chair-sitting data of the users on the chair further includes synchronizing the collected pressure values and inclination values with Al vision data implemented by a plurality of cameras.

5. A computer program stored in a computer-readable recording medium that is coupled with a computer as hardware so as to be able to perform the method according to claim 1.

6. A posture correction apparatus (100) comprising:
a chair-sitting data collection unit (121) configured to collect chair-sitting data of users on a chair (300);
a data cluster construction unit (122) configured to construct a user-specific data cluster based on the collected chair-sitting data;
an Al algorithm construction unit (123) configured to construct an Al algorithm for posture correction based on the constructed data cluster; and
a posture analysis unit (132) configured to provide information on posture correction based on the constructed Al algorithm and real-time chair-sitting data.

7. The posture correction apparatus (100) according to claim 6, further comprising:
an Al algorithm update unit (124) configured to analyze improvement items of the users using the constructed Al algorithm and update the Al algorithm based on the analyzed improvement items.

8. The posture correction apparatus (100) according to claim 6, wherein
the chair-sitting data collection unit (121) is further configured to collect pressure values and inclination values from a pressure sensor (340) and a gyro sensor (330) attached to the chair (300).

9. The posture correction apparatus (100) according to claim 8, wherein
the chair-sitting data collection unit (121) is further configured to synchronize the collected pressure values and inclination values with Al vision data implemented by a plurality of cameras.

10. A posture correction system (10) comprising:
the posture correction apparatus (100) according to claim 6; and
a user terminal (200) communicably coupled with the posture correction apparatus (100).
